# EUROPEAN PATENT SPECIFICATION

(11) **EP 2 699 316 B1**
(45) Date of publication and mention of the grant of the patent: **01.06.2016**
(21) Application number: 12713966.5
(22) Date of filing: 05.04.2012
(51) Int. Cl.: A61N 7/02, A61N 7/00, A61B 18/00

(54) **TRANSOESOPHAGEAL DEVICE USING HIGH INTENSITY FOCUSED ULTRASOUND FOR CARDIAC THERMAL ABLATION**
TRANSÖSOPHAGEALE VORRICHTUNG MIT HOCHINTENSIVEM, FOKUSSIERTEM ULTRASCHALL ZUR KARDIALEN THERMISCHEN ABLATION
DISPOSITIF TRANSOESOPHAGIEN UTILISANT DES ULTRA-SONS FOCALISÉS DE HAUTE INTENSITÉ POUR UNE ABLATION THERMIQUE CARDIAQUE

(30) Priority: 05.04.2011 EP 11305392
(43) Date of publication of application: 26.02.2014
(73) Proprietor: INSERM - Institut National de la Santé et de la Recherche Médicale, 75013 Paris (FR); Université Claude Bernard Lyon 1, 69100 Villeurbanne (FR)
(72) Inventor: CHAPELON, Jean-Yves, 69424 Lyon Cedex 03 (FR); CONSTANCIEL, Elodie, 69424 Lyon Cedex 03 (FR); LAFON, Cyril, 69424 Lyon Cedex 03 (FR)
(74) Representative: Cabinet Plasseraud
(86) International application number: PCT/EP2012/056320
(87) International publication number: WO 2012/136786

(56) References cited:
- WO-A2-2006/032059
- DE-A1- 4 312 264
- US-A- 5 520 188
- US-A1- 2003 216 648
- US-A1- 2005 203 399
- US-A1- 2005 240 127
- US-A1- 2006 241 442
- US-A1- 2009 312 673

## Description

### TECHNICAL FIELD

The present invention relates to a transoesophageal device, and also to a system comprising said transoesophageal device and constituting an application for treating atrial fibrillation.

### BACKGROUND OF THE INVENTION

The closest prior art is formed by documents US2005/240127 A1 and US2003/216648 A1.

Atrial fibrillation is the most frequent cardiac arrhythmia. As an example, in 2008, it affected more than 750.000 people in France and should affect near to two million by 2050, in majority elderly people (65-80 years old).

Atrial fibrillation would be responsible of 15% of cerebrovascular accidents in France.

This arrhythmia is characterised by a disorderly and quick electrical activity, a loss of the atrial mechanical function and a thromboembolic risk. The resulting quick and irregular ventricular activity can be responsible of cardiac insufficiency.

This arrhythmia is caused by anatomical or electrophysiological abnormalities of the myocardium.

A successful treatment for the cardiac arrhythmia often requires blocking or modifying the electrical pulse conduction path in cardiac muscle, thus to resynchronize the cardiac rhythms to normal pace. In this regard, Cox described the maze III procedure consisting in a series of incisions arranged in a maze-like pattern in the atria.

The maze III procedure is also called the "gold standard" procedure because it is very efficient. However, this procedure is not often used because of its complexity.

That is why, a simplified version of Maze III procedure called "Mini-Maze" procedure has been proposed by scientists.

The intervention to cardiac electrical pulse conduction path can be performed by open chest surgery.

Another alternative is the transvenous radio frequency (RF) current catheter thermal ablation, in which the RF catheter tip is inserted percutaneously into the heart chamber through an artery or vein.

Both of these methods are invasive. Moreover, the surgical procedure by RF has a moderated efficiency in the persistent forms of atrial fibrillation.

A less-invasive cardiac thermal ablation technique has been proposed in earlier studies, where focused ultrasounds are used to ablate the cardiac tissue.

High Intensity Focused Ultrasounds (HIFU) can penetrate deeply into tissue and produce large lesions by thermal mechanisms without requiring a direct contact between the probe and the targeted tissue.

However, the existing ultrasonic devices are positioned in direct contact with the epicardium (Epicor Cardiac Ablation System) or in an intracardiac manner ("balloon catheter", Meininger and al., Journal of Interventional Cardiac Electrophysiology, vol.8, p141-148, 2003)*.*

There is a real need to develop apparatus for treating atrial fibrillation which are still less invasive.

Xiangtao and al. (IEEE Transactions on Ultrasonics, Ferroelectrics, and Frequency control, vol.53, N°6, p1138-1149, 2006*)* and Werner and Al. (Ultrasound in Medicine and Biology, vol.36 n °5, p752-760, 2010*)* proposed a less invasive method by using a 2D focused ultrasound phased array transducer to carry out a non-invasive transesophageal cardiac thermal ablation.

Although the ideal design of a transducer, when attempting to sweep a focal zone in three dimensions, is a 2D phased array transducer (since it allows an electronic focusing and an electronic deflection of the HIFU beam), the design of a 2D phased array transducer is complexed and its multiple elements involve many interconnections leading to a difficult manufacturing process.

Furthermore, the electronic deflection of the HIFU beam does not allow performing all the "Mini-Maze" procedure: a mechanical rotation is still necessary.

Analysis of the prior art leads to the observation that there is a need for a transesophageal HIFU device able to perform the "Mini-Maze" procedure mini-invasively (i.e. transmural thermal ablations on myocardium according to Cox's pattern), being economic, reliable, suitable for a transesophageal use, and not requiring many interconnections.

The transesophageal HIFU device should also be able to visualize in real time the targeted tissue to be treated and should not have an imposed imaging plane so as to visualize the heart in any plane.

The Applicant found that a HIFU therapy transducer being a 1D phased array annular transducer, said therapy transducer being in combination with a central multiplane imaging transducer is capable of meeting these needs.

### SUMMARY OF THE INVENTION

The object of the present invention is to provide a transoesophageal device (hereafter a "probe"), comprising:
- a piezoelectric therapy transducer, hereafter therapy transducer having an acoustic axis BB', and
- an imaging transducer having an imaging plane,
said therapy transducer and imaging transducer being mounted in a head itself connected to a guide means wherein:
- the therapy transducer is formed by a truncated cup and has a spherical concave front face for emitting ultrasonic waves focused on a focal point, a rear surface, a length d₁, a width t and is a 1D annular phased array transducer, and
- the imaging transducer is a multiplane transducer configured to rotate about the acoustic axis of the therapy transducer, whereby the focal point of the therapy transducer is comprised in the imaging plane of the imaging transducer, said imaging transducer being fixed to the therapy transducer.

According to an embodiment of the present invention, the probe of the invention is characterized by the fact that:
- the spherical concave front face includes an open window for mounting the imaging transducer;
- the imaging transducer is centred relatively to the therapy transducer, and
- the imaging transducer is a 1D phased array imaging transducer.

Another object of the present invention is a therapeutic system comprising a probe module including the probe according to the present invention, a treatment module and a command module.

According to an aspect of the invention, the probe module further comprises a cooling system to prevent an overheating of the therapy transducer, to cool down the inner surface of the esophagus, and to assure the acoustic coupling.

According to another aspect of the invention, the system further comprises an electrocardiogram system (ECG) for receiving vital sign information from the patient in order to synchronize the HIFU delivery according to the cardiac cycle.

According to another aspect of the invention, the system further comprises a pre-diagnostic system in order to merge the preoperative images with the peroperative images.

According to another aspect of the invention, the system further comprises an electroanatomical mapping system to visualize atrial activation and so to detect abnormal electrical conduction paths.

### DESCRIPTION OF DRAWINGS

Embodiments of the invention are described by way of example with reference to the accompanying drawings, in which:
- Figure 1 is a schematic front view of a probe according to the present invention;
- Figure 2 is a sectional view of the probe of figure 1 along AA' cut plane;
- Figure 3 is a system block diagram for the therapeutic system according to the present invention;
- Figure 4 is a scheme of a cooling system according to the invention;
- Figures 5 represent respectively (a) a transversal section of VHP (Visual Human Project) and (b) its numerical model (dotted circle: zoom on the mirror and flipped heart, plain circle: a pulmonary vein (PV)). A lesion (L) is shown in dark.
- Figures 6 are (a) the sagittal segmented section of the top part of the left atrial isthmus and (b) the transversal segmented section of the top part of the left atrial isthmus.
- Figures 7a-d represent the pressure field maps for a natural focusing of 40 mm. a and b show YZ plane (transversal section) where Y axis represents the width of the transducer and Z axis represents the acoustic axis BB'. (a: simulated pressure field map ; b: measured pressure field map) ; c and d show XZ plane (transversal section) where X axis represents the length of the transducer and Z axis represents the acoustic axis BB' (c: simulated pressure field map ; d: measured pressure field map).
- Figures 8a-d represent the pressure field maps for a focusing of 17 mm. a and b show YZ plane (transversal section) where Y axis represents the width of the transducer and Z axis represents the acoustic axis BB'. (a: simulated pressure field map ; b: measured pressure field map). C and d show XZ plane (transversal section) where X axis represents the length of the transducer and Z axis represents the acoustic axis BB' (c: simulated pressure field map ; d: measured pressure field map).
- Figures 9a-d represent the pressure field maps for a focusing of 55 mm. a and b show YZ plane (transversal section) where Y axis represents the width of the transducer and Z axis represents the acoustic axis BB'. (a: simulated pressure field map ; b: measured pressure field map) ; c and d show XZ plane (transversal section) where X axis represents the length of the transducer and Z axis represents the acoustic axis BB' (c: simulated pressure field map ; d: measured pressure field map).
- Figures 10a-d represent the pressure field maps for a focusing of 31 mm. a and b show YZ plane (transversal section) where Y axis represents the width of the transducer and Z axis represents the acoustic axis BB' (a: simulated pressure field map ; b: measured pressure field map) ; c and d show XZ plane (transversal section) where X axis represents the length of the transducer and Z axis represents the acoustic axis BB' (c: simulated pressure field map ; d: measured pressure field map).
- Figures 11 show sections of X-rays Computed Tomography (CT) images which represent imaging plane possibilities (a: transversal section of the top part of the left atrium ; b: transversal and sagittal sections of the top part of the left atrium; c: transversal and sagittal sections of the top part of the left atrium and 3D trachea representation; d: transversal and other sections of the top part of the left atrium and 3D trachea representation; e: transversal and optimal sections of the top part of the left atrium to measure the minimum distance between BB' and the trachea).

### DETAILED DESCRIPTION

In the art of the invention:
- a phased array transducer is a transducer cut up in multiple elements which are driven independently;
- a 1D transducer is a phased array transducer cut up in multiple elements in only one direction;
- a 1D annular phased array transducer comprises multiple concentric piezoelectric rings. Signals with different phase can be applied on each ring of the transducer;
- a 2D transducer is a phased array transducer cut up in the same number of elements in two directions;
- a multiplane transducer is a 1D transducer which can image multiple plans by a rotation (here around BB' axis) of the transducer. The rotation can be mechanical or electronic.

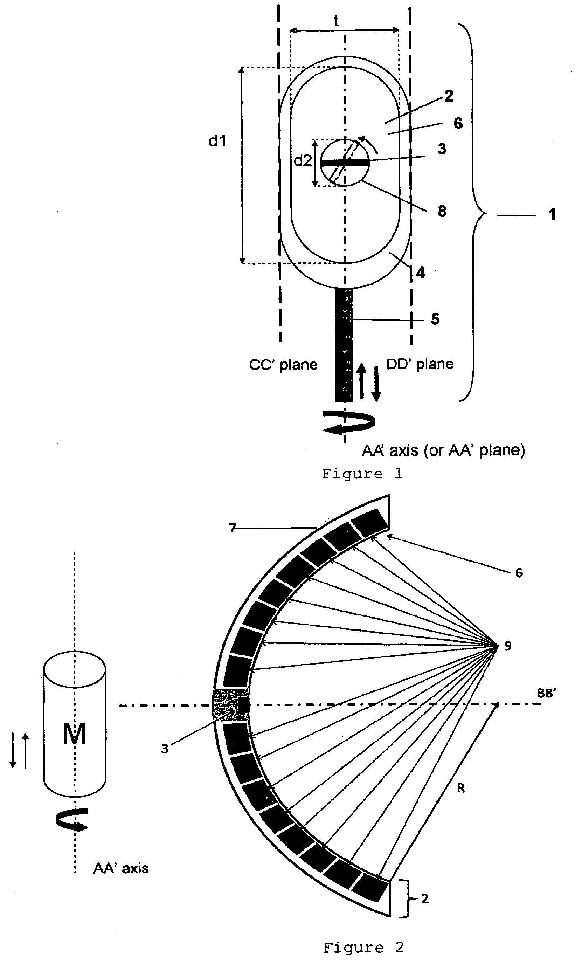

According to the invention, an orthonormal coordinate system is used in which,
- BB' axis is the acoustic axis of the therapy transducer;
- AA' plane is the plane which crosses longitudinally through the center of the probe as represented on figure 1;
- AA' axis is comprised in the AA' plane and represents the intersection of the AA' plane and a plane perpendicular to said AA' plane as represented on figure 1.

According to the invention, the therapy transducer is an ultrasound transducer which can be a piezoelectric transducer, a piezocomposite transducer or a CMUT (Capacitive Micromachined Ultrasound Transducer).

The object of the invention is a probe comprising a therapy transducer which is an 1D annular phased array transducer having a central imaging transducer which is a multiplane transducer. The "phased array" nature of the annular transducer allows the electronic focusing of the HIFU beam along the acoustic axis and thus can adjust the depth of the treatment relative to the HIFU source. The "1D" nature of the annular transducer does not require many interconnections since a few elements are involved.

Moreover, since the rotation inside the esophagus is a simple motion, there is no need for an electronic deflection (which is the case with 2D phased array transducers of the prior art): a simple rotation of the HIFU transducer inside the esophagus allows steering the HIFU beam.

The imaging transducer allows monitoring in real time the formation of the HIFU lesions to prevent the emergence of any secondary lesion.

The "multiplane" nature of the imaging transducer allows visualizing the heart in any plane since no imaging plane is imposed. The acoustic axis is always visible in any plane since it is also the rotation axis of the imaging transducer.

The "multiplane" nature of the imaging transducer enables also to perform a 3 Dimensions (3D) visualization of the heart.

The probe according to the invention is schematically represented on figure 1.

The probe 1 includes a therapy transducer 2 emitting HIFU which is an 1D annular phased array transducer and which comprises multiple concentric piezoelectric rings 9 (shown only on figure 2).

The probe 1 includes also an imaging transducer 3 directly integrated inside the therapy transducer 2.

This relative position between a therapy transducer and an imaging transducer is described in US 8,043,218.

Both of the imaging and therapy transducers are mounted in a head 4 itself connected to a flexible guide means 5.

The head 4 can be made in any material, preferably biocompatible, as long as this material is a good electrical insulator, a good thermal conductor and rigid enough for allowing safe insertion of the probe in the esophagus. Electrical insulation is required for preventing shortcuts between the faces of the elements. Thermal conduction allows removing excessive heating produced by the therapy transducer. The head must be MRI compatible for an eventual application inside an MRI.

According to the invention, the probe is inserted into the esophagus of the patient with the help of the guide means 5 and the displacements of the probe (i.e. rotation and translation) in the esophagus can be carried out by motorized means M (as represented on figure 2), for examples motorized positioning stages.

According to the invention, the probe can rotate around AA' axis and can translate along AA' axis (see figure 1).

According to the present invention, the therapy transducer is a 1D annular phased array transducer having a spherical front face and a rear face.

According to the invention, "spherical" means a concave shape having a radius of curvature R.

According to the present invention, the mean radius of curvature R is comprised from 30 to 60mm, preferably from 34mm to 44mm, and more preferably from 38mm to 42mm.

The 1D annular phased array transducer has an operating frequency between 1 and 5 MHz, preferably between 3 and 4 MHz.

According to another aspect of the invention, the 1D annular phased array transducer emits ultrasonic waves with a pulse duration located in the range from 0.5 to 10s, preferably from 2 to 7s, and more preferably from 4 to 6s, the ultrasonic waves being separated by a rest period located in the range from 2 to 40s, preferably from 5s to 30s, and more preferably from 10s to 20s.

According to another aspect of the invention, the 1D annular phased array transducer provides at its surface ultrasonic intensity from 4W/cm² to 12W/cm², preferably from 6W/cm² to 10W/cm².

The person skilled in the art will be able to choose a suitable pulse duration, rest period and ultrasonic intensity according to the distance between the 1D annular phased array transducer and the targeted tissues.

The person skilled in the art will also be able to control the treatment period according to the lesions appearing as one goes along on the peroperative images given by the imaging transducer.

The spherical concave shape of the front face of the therapy transducer 2 allows a first natural focusing. According to a feature of the invention, the therapy transducer 2 has a geometrical focal length corresponding to the radius R of the spherical front face 6 from 30mm to 60mm, preferably from 34 mm to 44 mm and more preferably from 38mm to 42mm.

Moreover, as shown in figure 2, the therapy transducer 2 comprises multiple piezoelectric rings 9 that can be controlled independently for moving electronically the focus (electronic focusing) along the acoustic axis BB' and thus adjusting the depth of the treatment. Therefore, the electronic focusing enables the displacement of the focal point around its natural location (imposed by the radius of curvature R).

According to an advantageous embodiment feature, the number of rings is selected from 6 to 20, preferably from 6 and 12, and more preferably is 8 rings.

In another aspect of the invention, the therapy transducer 2 is truncated such as a small width providing a narrow shape is obtained so as to facilitate the ingestion of the probe by the patient.

As shown on figure 2, the therapy transducer 2 is in the form of a cup with a concave shape. Said therapy transducer 2 has its opposite edges truncated along two planes CC' and DD' which are parallel to plane AA', each plane being preferably provided at an equal distance from plane AA' (figure 1).

In other terms, according to the front view of the probe represented on figure 1, after truncation, the therapy transducer 2 has an oblong form having a length d1 and a width t.

According to an embodiment, the ratio between d1 and t is comprised from 1.05 to 3.33, preferably from 1.31 to 2.66 and more preferably from 1.65 to 2.14 to allow enlarging the surface of the elements of the transducer and the focusing gain while favouring insertion of the probe in the esophagus.

According to a preferred embodiment, the width t is ranged from 12mm and 19mm, preferably from 14mm to 17mm and still preferably equal to 15 mm. According to an embodiment, the length d₁ of the therapy transducer is from 20mm to 40mm, preferably from 25mm to 32mm and more preferably from 28mm and 30mm.

The concave spherical front face 6 of the therapy transducer 2 includes an open window 8 suitable for mounting the multiplane imaging transducer 3.

According to a preferred embodiment of the invention, the window 8 has preferably a circular section with a diameter d₂ of the window selected from 8mm and 15mm and preferably from 10mm to 13mm.

According to the invention, the imaging transducer 3 is centred relatively to the therapy transducer 2 so that the focal point of the therapy transducer 2 is comprised in the imaging plane of the imaging transducer 3.

According to the invention, the imaging transducer 3 is a multiplane transducer which is allowed to turn from 0° to 180°.

The imaging transducer 3 can rotate from 0° to 180° around BB' axis (see figure 1 where a first position is represented in solid lines and a second position of said imaging transducer 3 is represented after rotation in dotted lines).

The imaging transducer rotation is independent of the probe rotation.

According to the invention, the rotation axis of the imaging transducer 3 corresponds to (i.e. coincides with) the acoustic axis BB' of the therapy transducer 2. The rotation axis of the imaging transducer is the axis BB'.

According to the invention, the imaging transducer 3 is an ultrasound imaging transducer.

The imaging transducer 3 is a 1D phased array transducer used for transesophageal echocardiography.

The imaging transducer can be selected among the TEE transducers.

According to a preferred embodiment, the imaging transducer is a Vermon transducer TEE cardiac PA5.0/64.

The imaging transducer 3 has a central frequency from 4 MHz to 7.5 MHz, preferably from 5 MHz to 6 MHz.

The combination of the therapy transducer 2 being an 1D annular phased array transducer 2 with the imaging transducer 3 being a multiplane transducer is very advantageous since the nature of the therapy transducer according to the invention allows the focal point to remain in the imaging plane whatever its orientation and the rotation of the imaging transducer 3 allows visualizing the heart in any plane.

Indeed, the probe can rotate around AA' axis and can translate along said AA' axis. The imaging transducer can independently of the displacements of the probe rotate from 0° to 180° around BB' axis.

Therefore, a probe according to the invention enables not only sagittal and transversal sections but also any others sections which include BB' axis so as to visualize all the tissues through which the HIFU pass. It is, for example, particularly interesting to visualize areas to be avoided, such as the trachea or lungs.

It also helps the practitioner to localize the areas to treat.

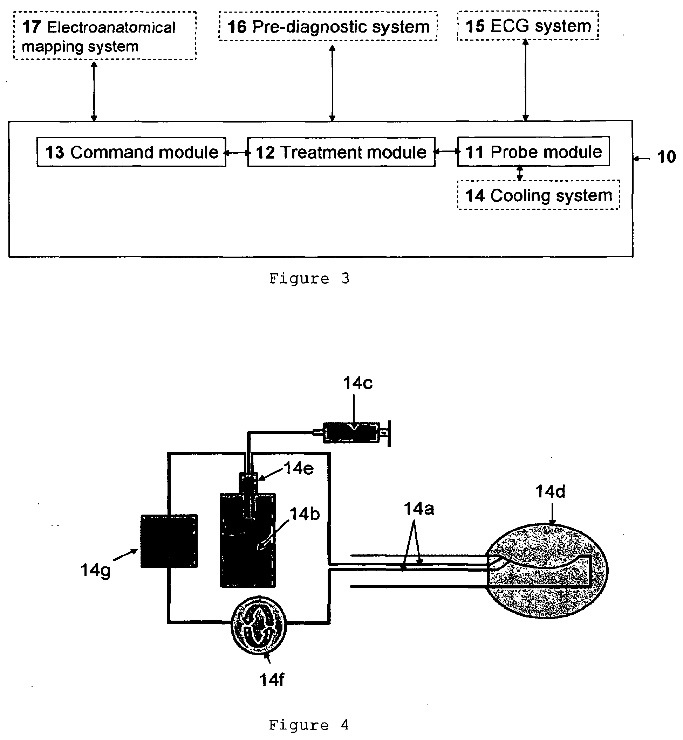

Figure 3 shows the therapeutic system 10 according to the invention comprising a probe module 11 including the probe according to the present invention, a treatment module 12 and a command module 13.

According to an aspect of the invention, the probe module further comprises a cooling system 14 which fulfils different functions such as the cooling of the therapy transducer, the cooling of the inner surface of the esophagus and the acoustic coupling.

As shown in figure 4, this cooling system 14 comprises two pipes 14a carrying out a circulation of water to cool the front face 6 of the therapy transducer 2. These pipes 14a are connected to a container 14b, closed hermetically, holding a quantity of degassed water at room temperature.

Cooled water can also be used to preserve the therapy transducer and the esophageus. A syringe 14c is used to fill and deflate from a distance a balloon 14d surrounding the therapeutic probe 1 which allows eliminating bubbles being initially present in the pipes 14a and the balloon 14d by trapping them under the hermetic cap 14e of the container. A peristaltic pump 14f carries out the circulation of water cooled by the heat exchanger 14g.

According to another aspect of the invention, the therapeutic system 10 further comprises an ECG system 15 so as to synchronize the HIFU delivery according to the cardiac cycle.

The man skilled in the art will be able to adapt the ultrasonic intensity of the therapy transducer 2, the pulse duration of the ultrasonic waves and the rest period according the cardiac cycle; the values of the operating frequency and the values of the central frequency of the imaging transducer being in the same range than ones used when the HIFU delivery is not synchronize with the cardiac cycle.

According to the invention, when an ECG system is coupled with the therapeutic system, the therapy transducer emits ultrasonic waves with a pulse duration located in the range from 0.2s to 1.2s, and preferably from 0.3s to 0.6s, the ultrasonic waves being separated by an extinction duration corresponding to the systole period, typically from 0.2s to 0.5s.

According to the present invention, the treatment module 12 may comprise a group generator/amplifier which applies signals with different phase on each piezoelectric ring 9 of the therapy transducer 2. The temporal phase difference between these signals is used to move the focus electronically. This group also allows controlling transmitted and reflected powers to see the coupling with the sonicated medium.

The command module 13 may include a central computer, in particular a computer containing control software, which software is preferably constituted by operator interface software and command management software.

The command module 13 allows adjusting values of acoustical parameters (signals with different phases to be sent to each ring 9 of the 1D annular phased array transducer, acoustic intensity, pulse duration) and sends data to the generator-amplifier.

According to another aspect of the invention, the therapeutic system 10 of the present invention is able to merge the peroperative images with the preoperative images and thus to drive automatically the therapy transducer 2 towards the tissue to be treated.

According to the invention, preoperative images means images acquired before operation.

According to the invention, peroperative images means images acquired during operation.

For this purpose, the therapeutic system 10 further comprises a pre-diagnostic system 16 and the command module 13 comprises software configured to target the zone to be treated on the preoperative images, to merge the preoperative images with the peroperative images and to focus automatically the beam of the therapy transducer towards these defined zones via data sent to the generator-amplifier 11.

The pre-diagnostic system 16 includes an imaging system for producing an image of the target tissue. Examples of imaging system include MRI system, scanner and ultrasounds system.

According to another aspect of the invention, the therapeutic system 10 further comprises an electroanatomical mapping system 17 to visualize atrial activation and so to detect abnormal electrical conduction paths.

The present invention relates also to a method for treating atrial fibrillation comprising the following steps:
(i) Optionally, acquiring preoperative images with the help of a pre-diagnostic system 16 on which the "Mini-Maze" procedure is drawn;
(ii) Inserting the probe 1 into the esophagus with the guide means 5;
(iii) Locating automatically the target to treat with the help of the imaging transducer 3 ;
(iv) Optionally, merging preoperative images with peroperative images with the help of a software included in the command module and targeting zones to treat;
(v) Moving the probe 1 automatically in the front of the target with the help of motorized means;
(vi) Adjusting automatically the acoustical parameters with the help of the command module 13 and sending them to the generator-amplifier;
(vii) Delivering HIFU shots with the help of the therapy transducer on the target;
(viii) Repeating the previous steps on a new area.

When the probe 1 is used with the cooling system 14, the balloon 14d surrounding the probe is inflated before step (iii), is deflated before step (v) and inflated after step (v).

The present invention relates also to a method for treating atrial fibrillation comprising the following steps:
(i) Optionally, acquiring preoperative images with the help of a pre-diagnostic system 16 on which the "Mini-Maze" procedure is drawn;
(ii) Inserting the probe 1 into the esophagus with the guide means 5;
(iii) Locating the area to treat with the help of the imaging transducer 3 ;
(iv) Moving the probe 1 in the front of the target with the guide means 5;
(v) Locating the target to treat on the ultrasound image.
(vi) Adjusting the acoustical parameters with the help of the command module 13 and sending them to the generator-amplifier;
(vii) Delivering HIFU shots with the help of the therapy transducer on the target;
(viii) Detecting a new area and repeating the previous steps.

The probe according to the invention is also suitable for treating other heart diseases such as heart valve diseases.

### EXAMPLES

### Example 1: Feasibility of transesophageal cardiac thermal ablation

The feasibility of transesophageal cardiac thermal ablation using a 1D annular phased array transducer according to the present invention was investigated in computer simulation studies.

### Simulation configuration:

Numerical simulations of a treatment of AF by transesophageal HIFU have been performed with a model previously developed *(Chavrier and al., 2000).*

Generated pressure maps are calculated from Rayleigh's integral. Then temperature maps are determined using Pennes' Bio Heat Transfer Equation (1948). Finally, the thermal dose inside tissues (in minute equivalent to 43°C) is calculated from the Sapareto and Dewey's formula (1984). Thereby a lesion is considered irreversible when the thermal dose is greater than or equal to 240 min i.e. 14400 s.

A realistic model of heart defined using the data of the Visible Human Project™ (VHP) (National Library of Medicine, Bethesda, MD) was taken. Particularly, male chest data, segmented by the Voxel-Man Group (University Medical Center Hamburg-Eppendorf), have been included in the model. Each voxel has got a volume of 1 mm³.

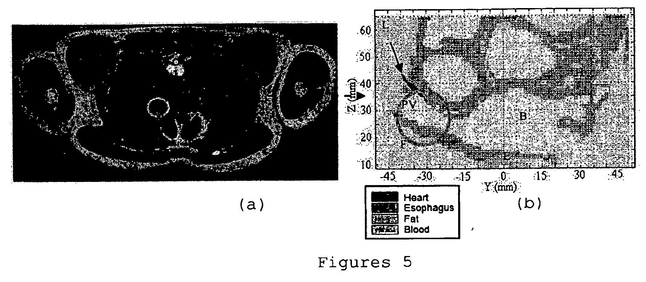

This model is made up of four types of tissue (Figure 5) with theirs own physical characteristics (TABLE 1):
- Heart (H) for myocardium and pericardium.
- Blood (B) for all veins and arteries but also cardiac cavities (atria and ventricles). Considering the important blood flow, temperature is maintained here at 37°C.
- Esophagus (E) is not present in the segmentation. So it has been defined manually as a cylinder with a 20 mm interior diameter and a 4 mm thick wall.
- Fat (F) for all the rest, to be in the most unfavorable case (high acoustic pressure attenuation to see all possible secondary lesions).

Esophagus is considered to be full of water to model the contents of the balloon surrounding the probe.

| **Tissues** | **Kₜ** | **C** | **T** | **Perf** | **ρ** | **α₀** | **b** |
|---|---|---|---|---|---|---|---|
| Water | 0.627 | 4188 | 37 | | 1000 | 0 | 1 |
| Eso. | 0.5 | 3600 | 37 | 7.11 | 1050 | 6.5 | 1 |
| Heart | 0.537 | 3600 | 37 | 14.2 | 1050 | 4 | 1 |
| Blood | 0.54 | 3770 | 37 | 1160 | 1050 | 1.6 | 1.22 |
| Fat | 0.2 | 3600 | 37 | 0 | 1050 | 9 | 1 |

TABLE 1: Tissue characteristics - Kₜ: Thermal conductivity (W.m⁻¹. °C⁻¹), C: specific heat capacity (J.kg⁻¹. °C⁻¹), T: Temperature (°C), Perf : Perfusion (kg.m⁻³.s⁻¹), ρ: Density of the tissue (kg.m⁻³), α₀ et b: coefficients of acoustic pressure attenuation α (Np.m⁻¹) such as α=α₀*f^{b}* with *f* the frequency (MHz).

The spatial resolution of the model is 0.75 mm and the temporal resolution is 0.2 s.

HIFU probe characteristics are given in the following TABLE 2.

| **Characteristics** | |
|---|---|
| d1 (mm) | 30 |
| t (mm) | 15 |
| d2 (mm) | 12 |
| R (mm) | 40 |
| Ring number | 8 |
| Frequency of the therapy transducer (MHz) | 3 |

Sequences of shots were defined and acoustical parameters of the transducer were chosen to recreate the procedure "mini-Maze" and minimize the number of secondary lesions.

The therapy transducer was emitting ultrasonic waves with a pulse duration located in the range from 0.5 to 10s, the ultrasonic waves being separated by a rest period located in the range from 2s and 40s.

### Summary of the results:

The proposed geometry allows performing precise lesions 17 to 50 mm far from the therapy transducer, i.e. really near the esophagus but also in the pulmonary vein region. Indeed, Figure 5b represents a transversal section showing the capacity to produce lesions (L) far from the esophagus (E), behind the pulmonary vein (PV) without creating secondary lesion on the esophagus.

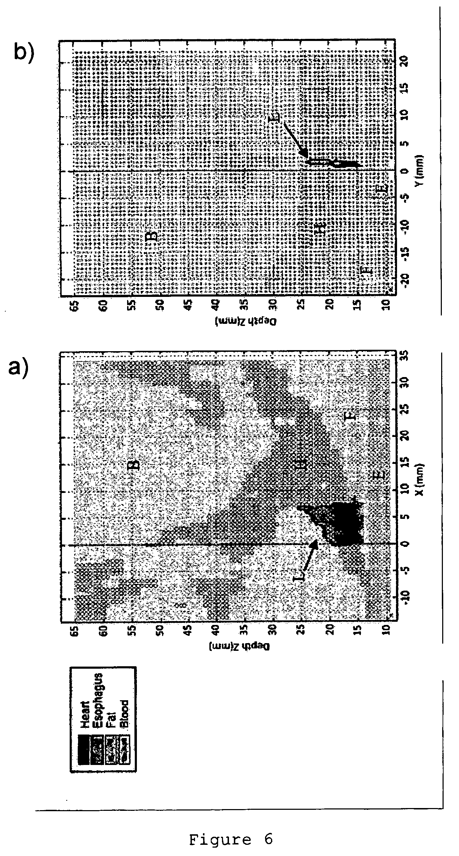

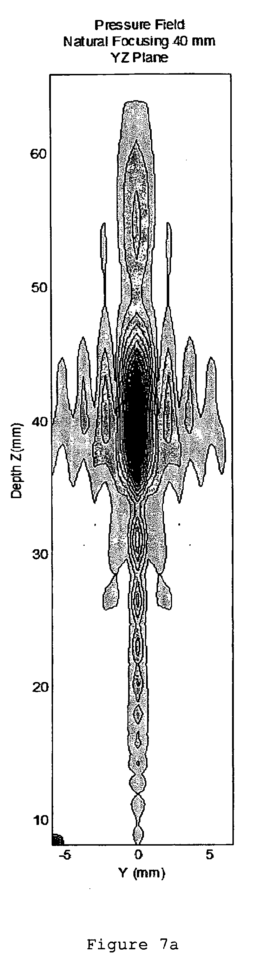

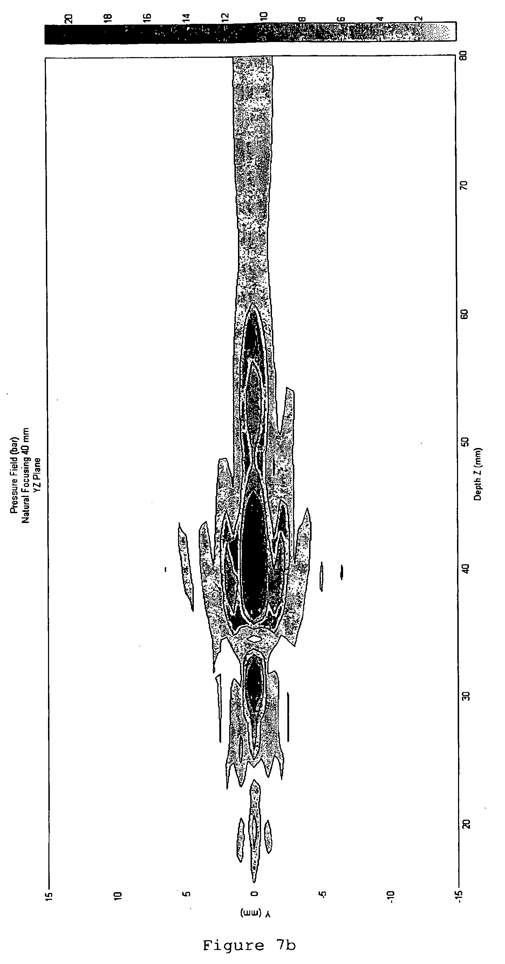

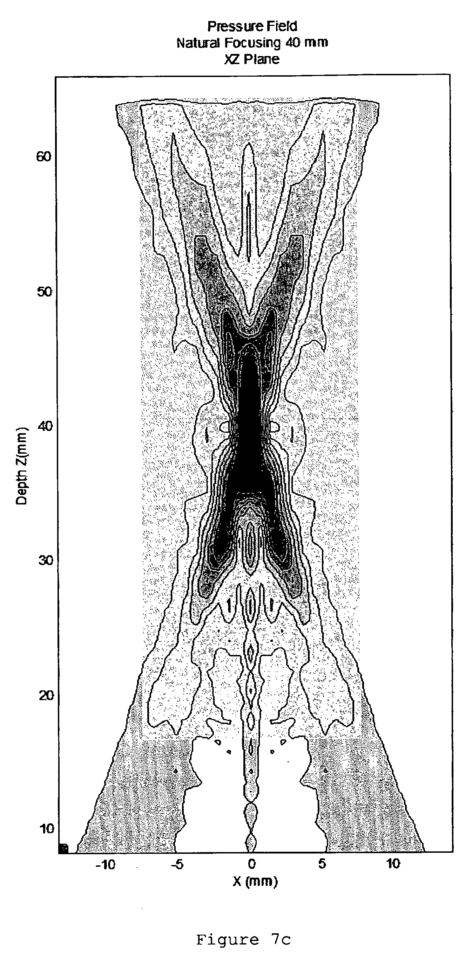

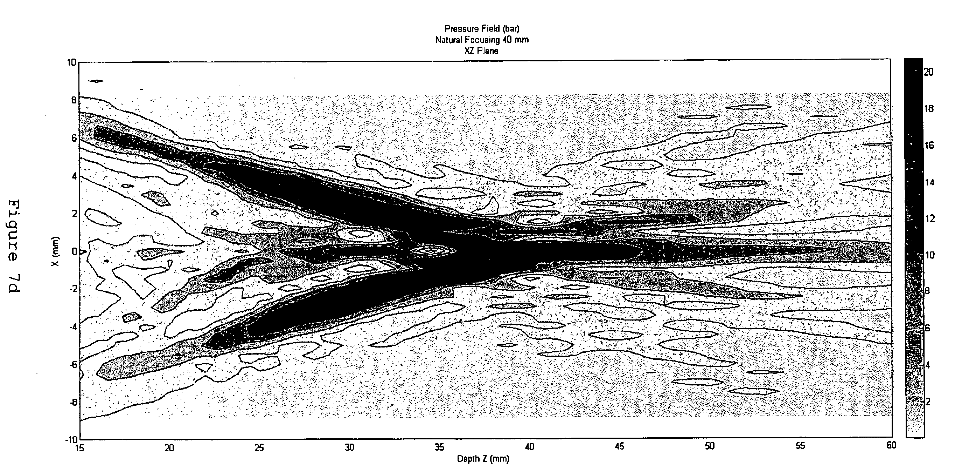

Moreover, no secondary thermal lesion on the esophagus was created thanks to the thinness of the lesions. Indeed, figures 7 representing the sagittal section of the top part of the left atrial isthmus shows the capacity to produce lesions (L) near the esophagus (E) without creating secondary lesion on the esophagus and representing (b) the transversal section shows the thinness of the lesions (2-3 mm wide) which are transmural and linear. A thermal lesion (L) was induced in the cardiac wall as it appears on figure 6a and 6b.

### EXAMPLE 2: Pressure field maps

Based on the method of the example 1, simulations have been performed with the following characteristics:
- d1 = 30 mm,
- d2 = 12 mm,
- t= 14 mm,
- R = 40 mm,
- Frequency of the therapy transducer = 3MHz,
- Number of rings = 8.

The conclusions of the example 1 are applicable to this geometry.

A prototype has been manufactured with these characteristics and pressure field maps according to different focusing (natural or electronic) have been performed.

These maps were obtained by motorized motion of a hydrophone (HGL-0200, Onda Corporation, Sunnyvale, CA, USA) in a tank of degassed water.

Two planes have been acquired. XZ plane is the AA' plane and YZ plane is the BB' plane.

First, pressure field maps have been performed at 40 mm focusing (Figure 7). This focusing is due to the transducer spherical shape. So this is the natural configuration. It offers the largest focal spot for the minimum number of secondary lobes.

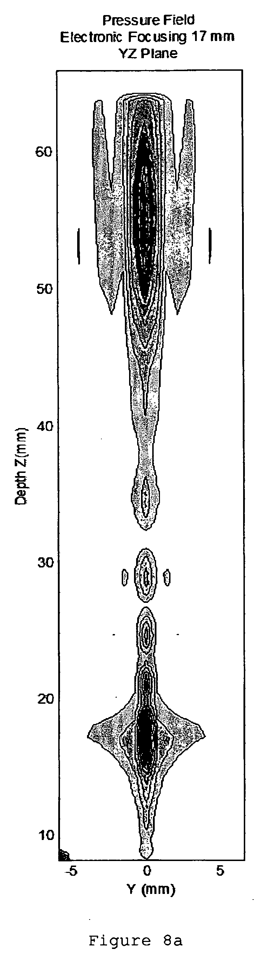

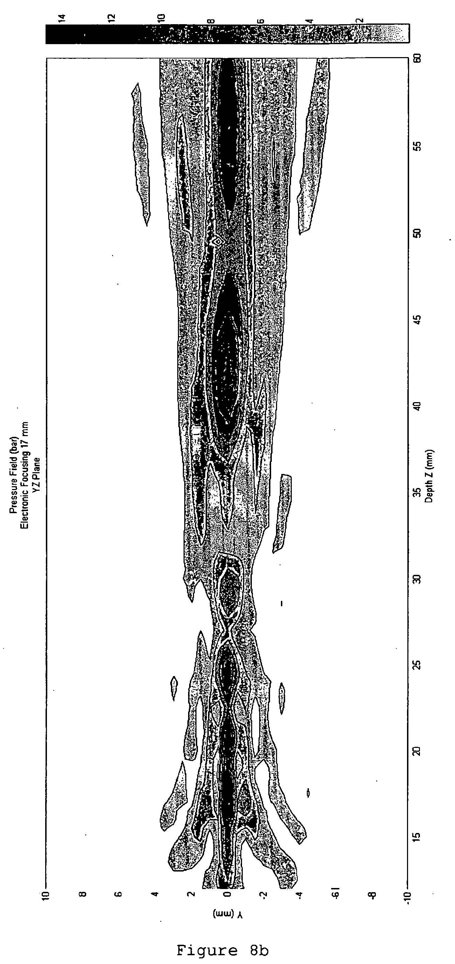

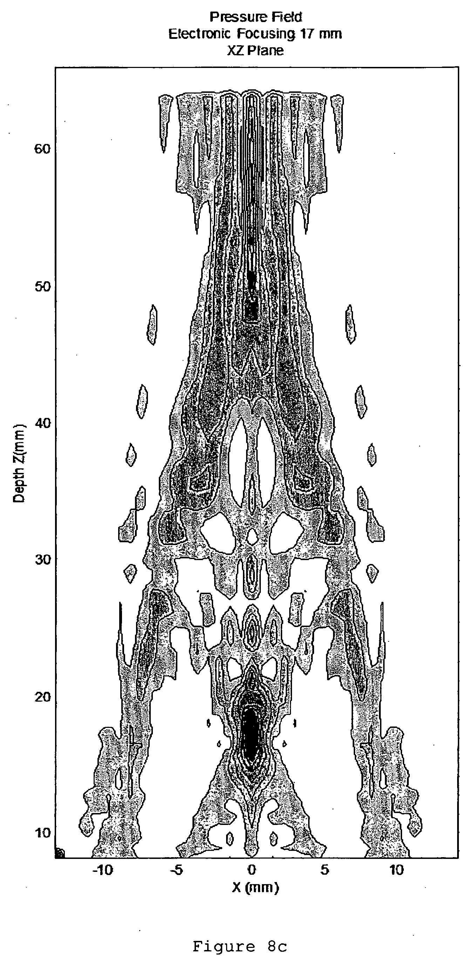

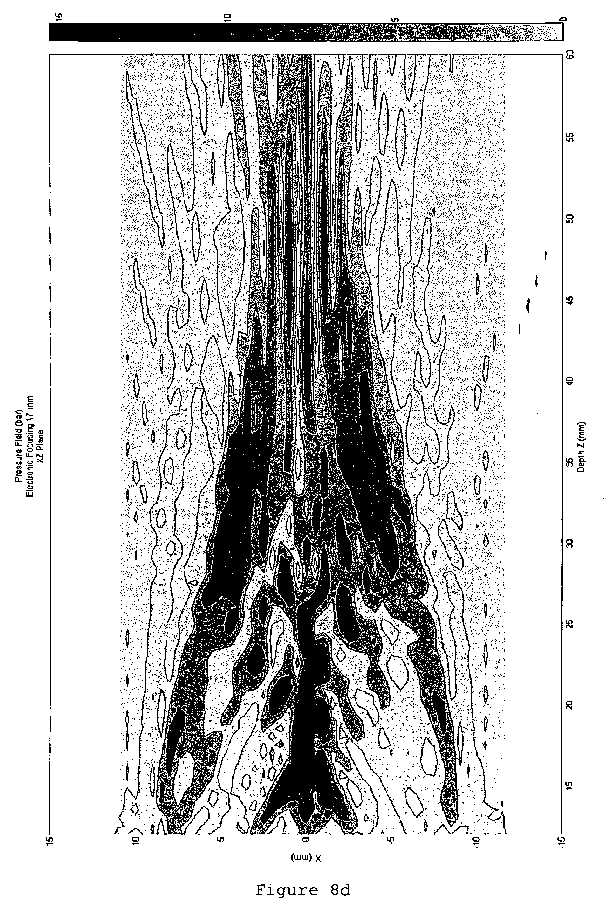

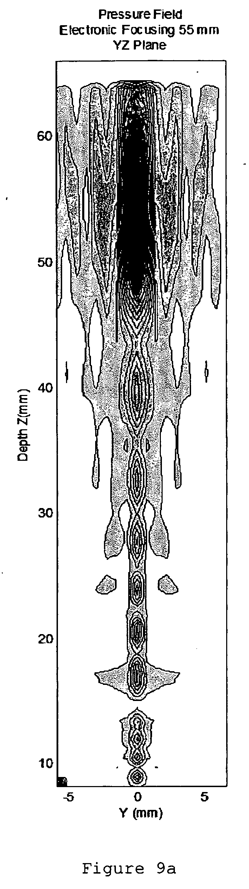

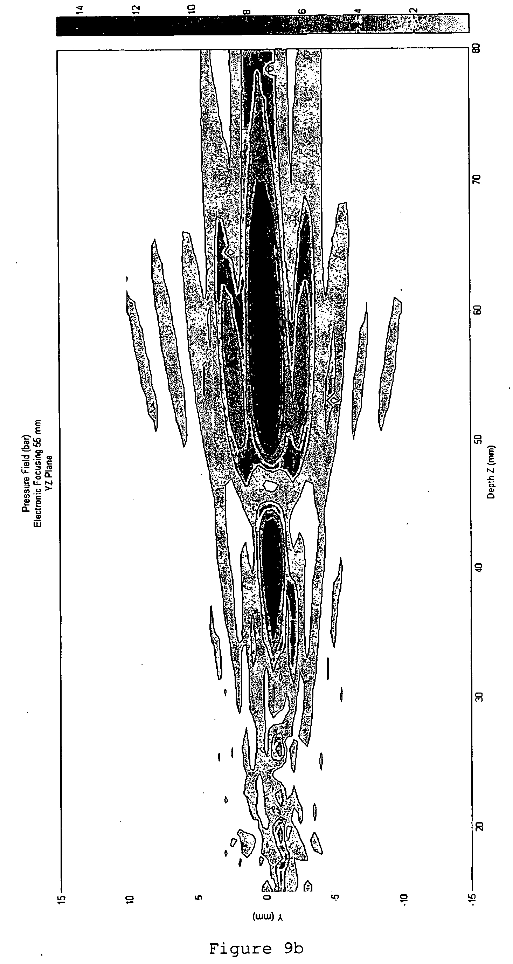

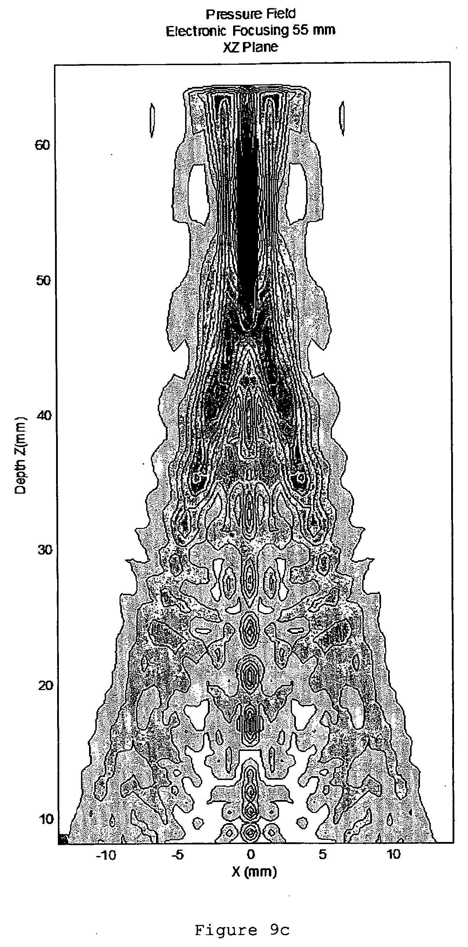

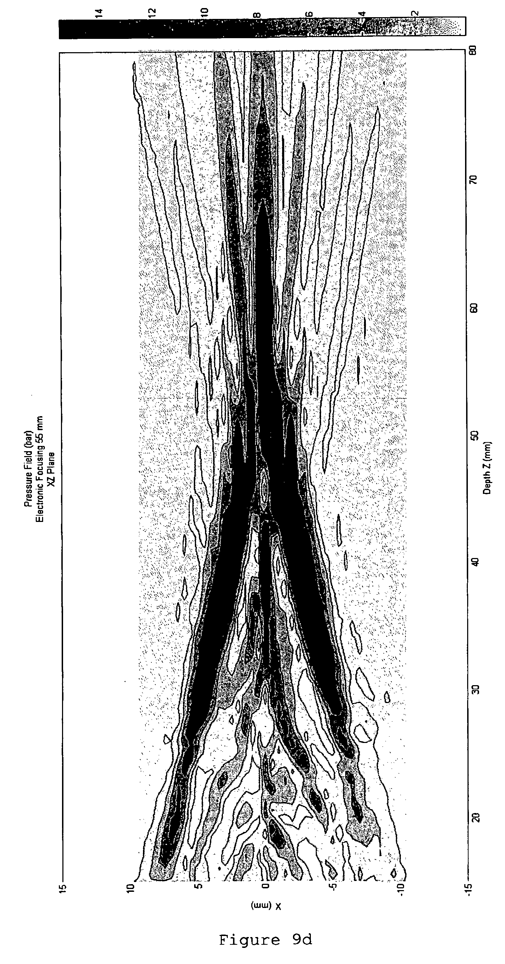

Then, the electronic focusing capacity has been tested. The extrema of the range of focusing are represented. 17 mm is the nearest focusing to the transducer (Figure 8) and 55 mm is the farthest (Figure 9).

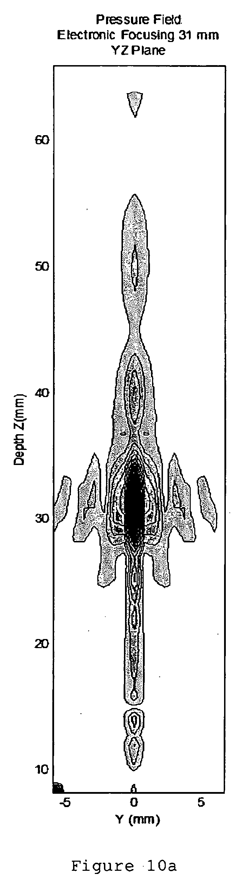

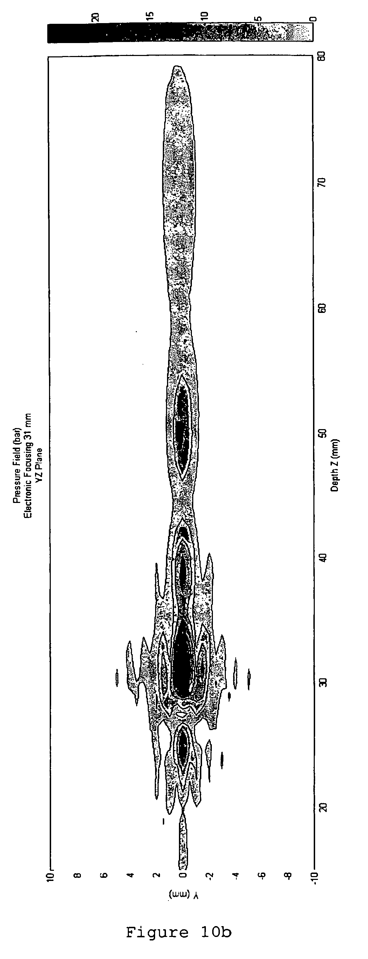

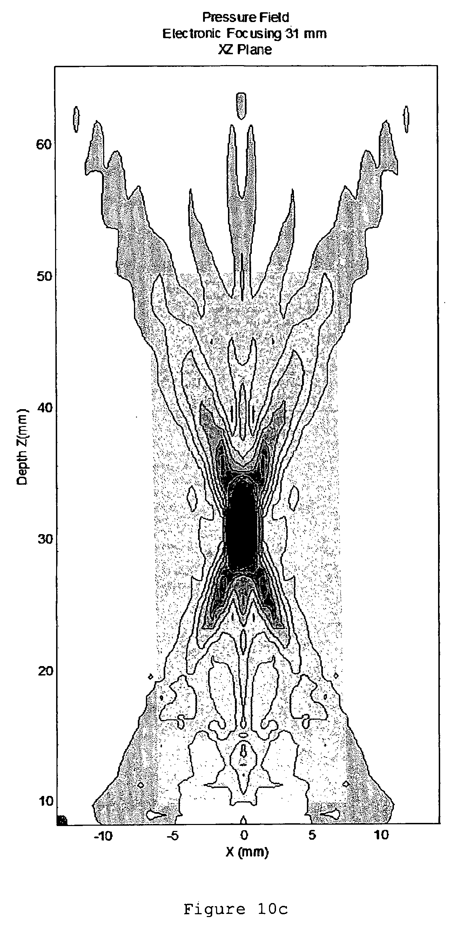

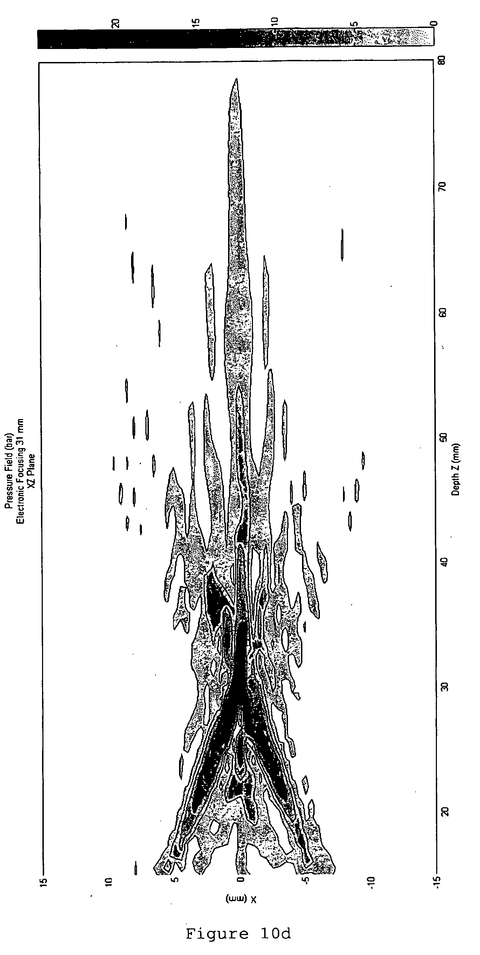

Finally, an intermediate focusing at 31 mm is given (Figure 10).

To interpret these maps, they are compared with simulated pressure field maps obtained in water with the model of the example 1.

For each figure, a and c are the simulated pressure field maps in respectively YZ plane and XZ plane. b and d are the measured pressure fields maps in respectively YZ plane and XZ plane.

The measured pressure field maps show that this first prototype is able to focus in a range between 17 mm and 55 mm.

These maps are relatively similar to the simulated ones with no big difference in the location of the secondary lobes.

So a prototype exhibiting the technical features of the present invention reproduces similar pressure field maps to the simulated ones.

### Example 3 : Multiplane imaging transducer

This example presents the point of having a multiplane imaging transducer instead of a biplane or a monoplane transducer. Here, CT images are used instead of ultrasound images.

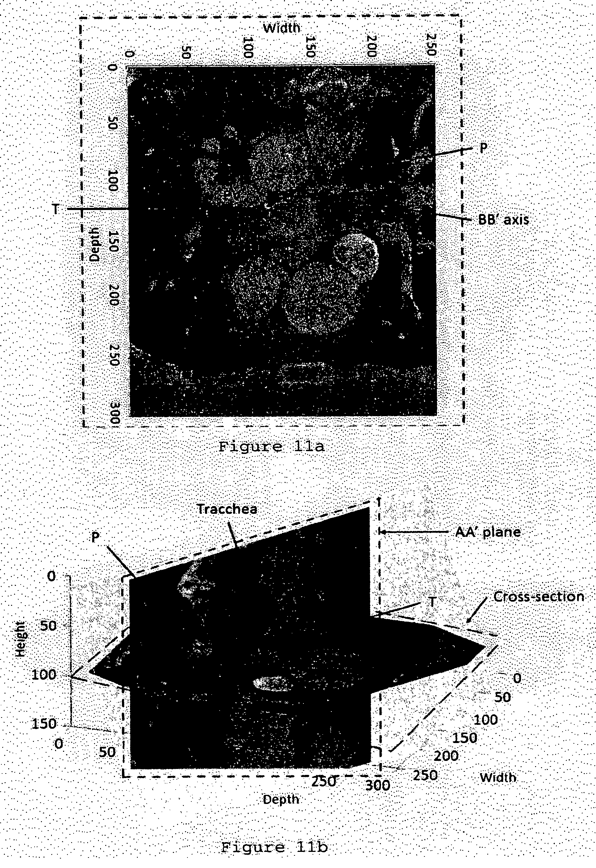

Figure 11a illustrates a cross-section of the left atrium upper part where a lesion has to be performed. The probe (P), the target (T) to treat and the acoustic axis BB' are represented on this figure. No particular obstacle for ultrasound propagation seems to be visible.

Figure 11b illustrates the same cross-section but also the AA' plane which can be visible with a biplane transducer. In this AA' plane trachea can be located and seems to be at a sufficient distance (d) from T.

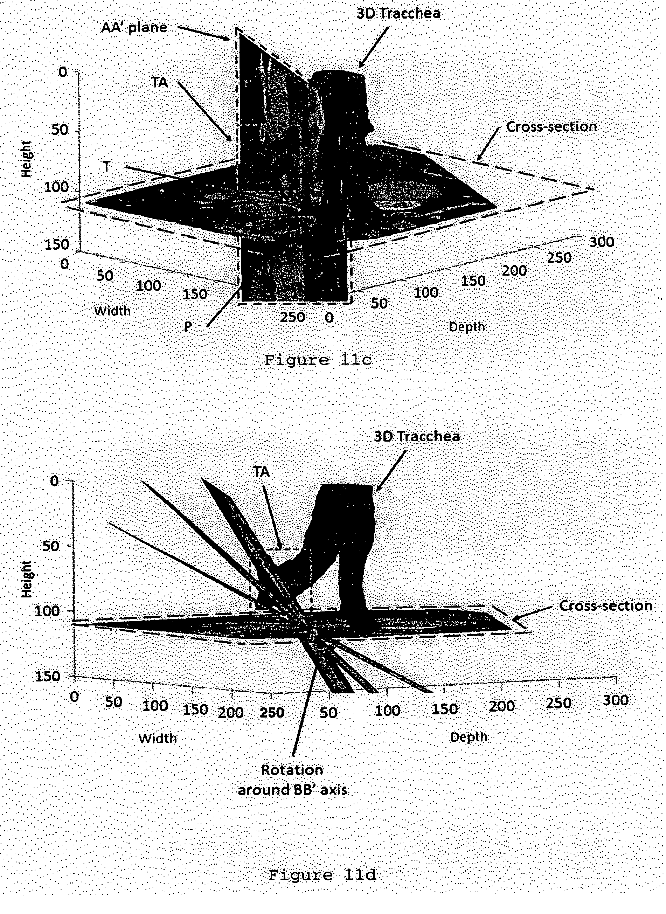

Figure 11c shows the two planes but also the 3D trachea representation which has been obtained by segmentation of the CT images. The minimum distance between the trachea area (TA) and T cannot be measured with a biplane transducer without a rotation of the entire probe around the AA' axis.

Figure 11d shows the capacity of a multiplane transducer to image all the TA area.

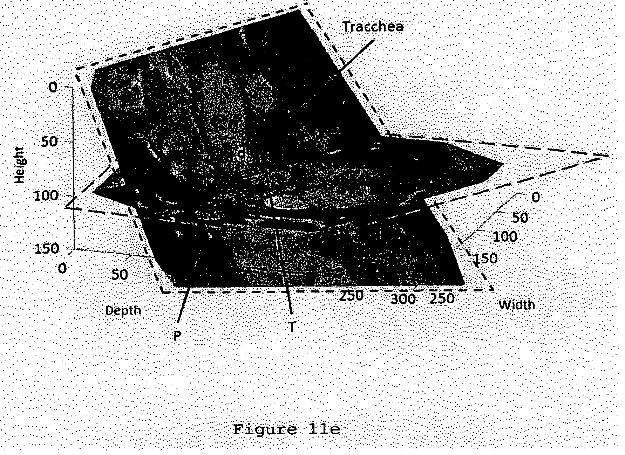

Figure 11e shows the plane where the distance between the TA area and T is minimum (dₘᵢₙ). This plane can be obtained using the multiplane transducer.

A precise location of structures like trachea or lungs which are full of air is really important for the success of the treatment and the safety of the patient. Because of the respiratory movement and the beating heart during the procedure, when these structures are potentially near the acoustic axis, they have to be localizable at any moment of the treatment without having to move the probe.

So using a multiplane transducer is better than using a biplane or a monoplane transducer, because these structures have complex shapes.

## Claims

1. A probe (1), comprising:
- a piezoelectric therapy transducer (2) having an acoustic axis BB', and
- an imaging transducer (3) having an imaging plane, said therapy transducer (2) and imaging transducer (3) being mounted in a head (4) itself connected to a guide means (5), wherein:
- the therapy transducer (2) is in a form of an ablong truncated cup and has a spherical concave front face (6) for emitting ultrasonic waves focused on a focal point, a rear surface (7), said therapy transducer (2) being truncated along two parallel planes, whereby a length d₁ and a width t<d₁ are provided ;
- the therapy transducer (2) is a 1D annular phased array transducer ; and
- the imaging transducer (3) is a multiplane transducer configured to rotate about the acoustic axis of the therapy transducer (2), whereby the focal point of the therapy transducer (2) is comprised in the imaging plane of the imaging transducer (3), said imaging transducer being fixed to the therapy transducer (2).

2. A probe according to claim 1, wherein
- the spherical concave front face (6) includes an open window for mounting the imaging transducer (3);
- the imaging transducer (3) is centred relatively to the therapy transducer (2); and
- the imaging transducer (3) is a 1D phased array imaging transducer.

3. A probe according to claim 1 or 2, wherein the therapy transducer (2) comprises multiple piezoelectric rings (9), the number of rings being selected from 6 to 20.

4. A probe according to one of claims 1 to 3, wherein the length d₁ of the therapy transducer (2) is ranged from 20 mm to 40 mm.

5. A probe according to one of claims 1 to 4, wherein the width t is ranged from 12 mm to 19 mm.

6. A probe according to one of claims 1 to 5, wherein the spherical concave front face has a radius of curvature R from 30 mm to 60 mm.

7. A probe according to one of claims 1 to 6, wherein the therapy transducer (2) is configured to emit ultrasonic waves with a pulse duration located in the range from 0.5 s to 10 s, the ultrasonic waves being separated by a rest period located in the range from 2 to 40 s.

8. A probe according to one of claims 1 to 7, wherein the therapy transducer (2) provides at its surface peak ultrasonic intensity from 4 W/cm² to 12 W/cm².

9. A probe according to one of claims 1 to 8, wherein the therapy transducer (2) has an operating frequency from 1 MHz to 5 MHz.

10. A probe according to one of claims 1 to 9, wherein the imaging transducer (3) has a central frequency from 4 MHz to 7.5 MHz.

11. A therapeutic system (10), comprising a probe module (11) including the probe according to one of the claims 1-10, a treatment module (12) and a command module (13).

12. A therapeutic system (10) according to claim 11, wherein the probe module (11) includes a cooling system (14).

13. A therapeutic system (10) according to claim 11, including an ECG system (15).

14. A therapeutic system (10) according to claim 11, including a pre-diagnostic system (16) configured to collect preoperative images of the target tissues.

15. A therapeutic system (10) according to claim 14, wherein the pre-diagnostic system (16) includes an imaging system comprising an MRI scanner or an ultrasound imaging system.

16. A therapeutic system (10) according to claim 11, wherein the command module (13) includes software configured:
- to target the zones to be treated on preoperative images;
- to merge the preoperative images provided by the pre-diagnostic system (16) according to one of the claims 14-15 with the peroperative images provided by the imaging transducer (3); and
- to focus automatically the beam of the therapy transducer (2) towards the zones to be treated.

## Patentansprüche

1. Sonde (1), umfassend:
- einen piezoelektronischen Therapieübertrager (2) mit einer akustischen Achse BB', sowie
- einen Bildgebungsübertrager (3) mit einer Bildgebungsebene, wobei der Therapieübertrager (2) und der Bildgebungsübertrager (3) in einem Kopf (4) befestigt sind, der selbst mit einer Führungseinrichtung (5) verbunden ist, wobei:
- der Therapieübertrager (2) die Form eines länglichen gestutzten Bechers hat und eine sphärische konkave Vorderseite (6) zum Abstrahlen von Ultraschallwellen, die auf einen Brennpunkt fokussiert sind, und eine Rückseite (7) aufweist, wobei der Therapieübertrager (2) entlang zwei parallelen Ebenen gestutzt ist, wobei eine Länge d₁ und eine Breite t < d₁ vorhanden sind;
- der Therapieübertrager (2) ein 1D ringförmiger Stufenarrayübertrager ist; und
- der Bildgebungsübertrager (3) ein Mehrstellenübertrager ist, der eingerichtet ist, um um die akustische Achse des Therapieübertragers (2) zu rotieren, wobei der Brennpunkt des Therapieübertragers (2) von der Bildgebungsebene des Bildgebungsübertragers (3) umfasst ist, wobei der Bildgebungsübertrager am Therapieübertrager (2) befestigt ist.

2. Sonde gemäß Anspruch 1, wobei
- die sphärische konkave Vorderseite (6) ein offenes Fenster zum Befestigen des Bildgebungsübertragers (3) einschließt;
- der Bildgebungsübertrager (3) in Bezug auf den Therapieübertrager (2) zentriert ist; und
- der Bildgebungsübertrager (3) ein 1D Stufenarraybildgebungsübertrager ist.

3. Sonde gemäß Anspruch 1 oder 2, wobei der Therapieübertrager (2) eine Vielzahl von piezoelektrischen Ringen (9) umfasst, wobei die Anzahl der Ringe von 6 - 20 ausgewählt ist.

4. Sonde gemäß einem der Ansprüche 1 - 3, wobei die Länge d₁ des Therapieübertragers (2) im Bereich von 20 mm bis 40 mm liegt.

5. Sonde gemäß einem der Ansprüche 1 - 4, wobei die Breite t im Bereich von 12 mm bis 19 mm liegt.

6. Sonde gemäß einem der Ansprüche 1 - 5, wobei die sphärische konkave Vorderseite einen Kurvenradius r von 30 mm bis 60 mm hat.

7. Sonde gemäß einem der Ansprüche 1 - 6, wobei der Therapieübertrager (2) eingerichtet ist, um Ultraschallwellen mit einer Pulsdauer im Bereich von 0,5 s bis 10 s abzustrahlen, wobei die Ultraschallwellen durch eine Ruheperiode im Bereich von 2 bis 40 s voneinander getrennt sind.

8. Sonde gemäß einem der Ansprüche 1 - 7, wobei der Therapieübertrager (2) auf seiner Oberfläche eine Ultraschallspitzenintensität von 4 W/cm² bis 12 W/cm² bereitstellt.

9. Sonde gemäß einem der Ansprüche 1 - 8, wobei der Therapieübertrager (2) eine Arbeitshäufigkeit von 1 MHz bis 5 MHz hat.

10. Sonde gemäß einem der Ansprüche 1 - 9, wobei der Bildgebungsübertrager (3) eine Zentralhäufigkeit von 4 MHz bis 7,5 MHz aufweist.

11. Therapeutisches System (10) umfassend ein Sondenmodul (11), das die Sonde gemäß einem der Ansprüche 1 - 10, ein Behandlungsmodul (12) und ein Kommandomodul (13) umfasst.

12. Therapeutisches System (10) gemäß Anspruch 11, wobei das Sondenmodul (11) ein Kühlsystem (14) einschließt.

13. Therapeutisches System (10) gemäß Anspruch 11, das ein EKG-System (15) einschließt.

14. Therapeutisches System (10) gemäß Anspruch 11, das ein prädiagnostisches System (16), das eingerichtet ist, um präoperative Bilder vom Zielgewebe aufzunehmen, einschließt.

15. Therapeutisches System (10) gemäß Anspruch 14, wobei das prädiagnostische System (16) ein Bildgebungssystem umfasst, das einen MRI-Scanner oder ein Ultraschallbildgebungssystem einschließt.

16. Therapeutisches System (10) gemäß Anspruch 11, wobei das Kommandomodul (13) Software einschließt, die eingerichtet ist, um:
- auf die zu behandelnden Zonen in präoperativen Bildern ausgerichtet zu werden;
- die präoperativen Bilder, die durch das prädiagnostische System (16) gemäß einem der Ansprüche 14 - 15 bereitgestellt werden, mit den präoperativen Bildern, die durch den Bildgebungsübertrager (3) zur Verfügung gestellt werden, zusammenzulegen; und
- den Strahl der Therapieübertragers (2) automatisch auf die zu behandelnden Zonen zu fokussieren.

## Revendications

1. Sonde (1), comprenant :
- un transducteur de thérapie piézoélectrique (2) ayant un axe acoustique BB', et
- un transducteur d'imagerie (3) ayant un plan d'imagerie, ledit transducteur de thérapie (2) et ledit transducteur d'imagerie (3) étant montés dans une tête (4) elle-même reliée à un moyen de guidage (5), dans laquelle :
- le transducteur de thérapie (2) est sous la forme d'une coupelle tronquée oblongue et a une face avant concave sphérique (6) pour émettre des ondes ultrasoniques concentrées sur un point focal, une surface arrière (7), ledit transducteur de thérapie (2) étant tronqué le long de deux plans parallèles, moyennant quoi une longueur d₁ et une largeur t < d₁ sont fournies ;
- le transducteur de thérapie (2) est un transducteur à commande de phase annulaire 1D ; et
- le transducteur d'imagerie (3) est un transducteur multiplan configuré pour tourner autour de l'axe acoustique du transducteur de thérapie (2), moyennant quoi le point focal du transducteur de thérapie (2) est compris dans le plan d'imagerie du transducteur d'imagerie (3), ledit transducteur d'imagerie étant fixé au transducteur de thérapie (2).

2. Sonde selon la revendication 1, dans laquelle
- la face avant concave sphérique (6) comprend une fenêtre ouverte pour monter le transducteur d'imagerie (3) ;
- le transducteur d'imagerie (3) est centré par rapport au transducteur de thérapie (2) ; et
- le transducteur d'imagerie (3) est un transducteur d'imagerie à commande de phase 1D.

3. Sonde selon la revendication 1 ou 2, dans laquelle le transducteur de thérapie (2) comprend des anneaux piézoélectriques multiples (9), le nombre d'anneaux étant sélectionné de 6 à 20.

4. Sonde selon l'une des revendications 1 à 3, dans laquelle la longueur d₁ du transducteur de thérapie (2) va de 20 mm à 40 mm.

5. Sonde selon l'une des revendications 1 à 4, dans laquelle la largeur t va de 12 mm à 19 mm.

6. Sonde selon l'une des revendications 1 à 5, dans laquelle la face avant concave sphérique a un rayon de couverture R de 30 mm à 60 mm.

7. Sonde selon l'une des revendications 1 à 6, dans laquelle le transducteur de thérapie (2) est configuré pour émettre des ondes ultrasoniques ayant une durée d'impulsion se trouvant dans la plage de 0,5 s à 10 s, les ondes ultrasoniques étant séparées d'une période de repos se trouvant dans la plage de 2 à 40 s.

8. Sonde selon l'une des revendications 1 à 7, dans laquelle le transducteur de thérapie (2) présente à sa surface une intensité ultrasonique pic de 4 W/cm² à 12 W/cm².

9. Sonde selon l'une des revendications 1 à 8, dans laquelle le transducteur de thérapie (2) a une fréquence opérationnelle de 1 MHz à 5 MHz.

10. Sonde selon l'une des revendications 1 à 9, dans laquelle le transducteur d'imagerie (3) a une fréquence centrale de 4 MHz à 7,5 MHz.

11. Système thérapeutique (10), comprenant un module de sonde (11) incluant la sonde selon l'une des revendications 1 à 10, un module de traitement (12) et un module de commande (13).

12. Système thérapeutique (10) selon la revendication 11, dans lequel le module de sonde (11) comprend un système de refroidissement (14).

13. Système thérapeutique (10) selon la revendication 11, comprenant un système d'ECG (15).

14. Système thérapeutique (10) selon la revendication 11, comprenant un système de pré-diagnostic (16) configuré pour collecter des images préopératoires des tissus cibles.

15. Système thérapeutique (10) selon la revendication 14, dans lequel le système de pré-diagnostic (16) comprend un système d'imagerie comprenant un scanner IRM ou un système d'imagerie ultrasonique.

16. Système thérapeutique (10) selon la revendication 11, dans lequel le module de commande (13) comprend un logiciel configuré :
- pour cibler les zones devant être traitées sur des images préopératoires :
- pour fusionner les images préopératoires fournies par le système de pré-diagnostic (16) selon l'une des revendications 14-15 avec les images préopératoires fournies par le transducteur d'imagerie (3) ; et
- pour concentrer automatiquement le faisceau du transducteur de thérapie (2) vers les zones devant être traitées.
